Europäisches Patentamt

European Patent Office

Office européen des brevets

(19)

(11) Publication number: **0 061 253**
**A2**

(12) **EUROPEAN PATENT APPLICATION**

(21) Application number: **82301193.7**

(22) Date of filing: **09.03.82**

(51) Int. Cl.$^3$: **C 12 N 15/00**
C 12 P 17/18, C 12 N 1/20
//C12R1/465

(30) Priority: **18.03.81 GB 8108528**

(43) Date of publication of application:
**29.09.82 Bulletin 82/39**

(84) Designated Contracting States:
**BE CH DE FR GB IT LI NL**

(71) Applicant: **BEECHAM GROUP PLC**
**Beecham House Great West Road**
**Brentford Middlesex(GB)**

(72) Inventor: **Makins, John Frank**
**11 Onley Street**
**Norwich(GB)**

(72) Inventor: **Holt, Geoffrey**
**Chantemerle 13c Priestlands Park Road**
**Sidcup Kent, DA15 7HR(GB)**

(74) Representative: **Hesketh, Alan, Dr. et al,**
**European Patent Attorney Beecham Pharmaceuticals**
**Great Burgh Yew Tree Bottom Road**
**Epsom, Surrey, KT18 5XQ(GB)**

(54) Process for the transfer of genetic material into actinomycete cells, prepared cells and method of culturing these cells and isolating a metabolite.

(57) A process for the transfer of genetic material into Actinomycete cells, which comprises entrapment of the genetic material within a lipid vesicle and thereafter fusing the lipid vesicle with an osmotically fragile Actinomycete cell.

EP 0 061 253 A2

Croydon Printing Company Ltd.

TITLE MODIFIED
see front page

## BIOLOGICAL TREATMENT

This invention relates to a novel process for the transfer of genetic material into bacteria, and in particular to lipid vesicle mediated transformation of bacteria.

The application of genetics for strain improvement in industrial micro-organisms has recently been reviewed by M. Kikuchi in Biotechnology and Bioengineering Vol XXII Suppl 1. 195-208, 1980. It is known to effect genetic changes in micro-organisms by exposing micro-organisms to radiation, such as ultra violet radiation or X-ray radiation or by exposure to chemical mutagens; these types of genetic manipulation produce random changes in the genetic material.

A further known process for transferring genetic material between cells is protoplast fusion. A protoplast is a structure in which the cell wall has been partially or completely removed by a chemical or biological method leaving an intact cell membrane and cell contents. Protoplast fusion allows the transfer of genetic material between two or more protoplasts, which are then regenerated and cultured, and the resulting culture examined for recombinants. A disadvantage of the protoplast fusion method is that in addition to the desired genetic material being transferred, other pieces of genetic material may be transferred between the protoplasts leading to undesired properties in the regenerated cells. A further disadvantage of protoplast fusion is the necessity for the host and the donor protoplasts to be derived from compatable strains.

The present invention provides a process for the transfer of genetic material into Actinomycete cells, which comprises entrapment of the genetic material within a lipid vesicle and thereafter fusing the lipid vesicle with an osmotically fragile Actinomycete cell.

The genetic material may be completely entrapped within a lipid vesicle, or may be incompletely entrapped or associated with a lipid vesicle. Any genetic material not completely entrapped is also transferred into Actinomyete cells upon fusing the lipid vesicle with an osmotically fragile Actinomycete cell.

Suitable osmotically fragile actinomycete cells include protoplasts.

The invention also relates to novel Actinomycete cells produced from the transformations of the above process, and to colonies of cells cultured therefrom.

- 3 -

When used herein the term genetic material includes both DNA and RNA.  Suitably the genetic material is DNA     such as, for example, chromosomal, phage or plasmid DNA.  Most suitably the genetic material is chromosomal DNA.

The lipid vesicle may suitably be a phospholipid vesicle or be derived from actinomycetes.  Fusion of the lipid vesicle with the bacterial cell or protoplast may suitably be promoted by treatment with polyethylene glycol.  The polyethylene glycol will suitably have a molecular weight of 1000 to 6000, preferably about 1000.

The Actinomycete cells may be a wild type or a mutant thereof.

When used herein the term 'mutant' includes any mutant strain which arises spontaneously or through the effect of an external agent, whether that agent is applied deliberately or otherwise.  Suitable methods of producing mutant strains include those outlined by H.I. Adler in Techniques for the Development of Micro-Organisms in 'Radiation and Radioisotopes for Industrial Micro-Organisms', Proceedings of a Symposium  Vienna, 1973, page 241, International Atomic Energy Authority and these include:

i)    Ionising radiation (such as X- and $\gamma$-rays), uv light, uv light plus a photo-sensitizing agent (such as 8-methoxypsoralen), nitrous acid, hydroxylamine, pyrimidine base analogues (such as 5-bromouracil), acridines, alkylating agents (such as mustard gas or ethyl methane-sulphonate), hydrogen peroxide, phenols, formaldehyde and heat.

ii) Genetic techniques such as recombination, transformation, transduction, lysogenisation, lysogenic conversion and selective techniques for spontaneous mutants.

Suitably the Actinomycete cells are of the family Streptomycetaceae; preferably Streptomyces, such as for example S. coelicolor, S. clavuligerus, S. olivaceous, S. katsurahamanus, Streptomyces P6621, S. jumonjinensis and S. lividans; and in particular clavulanic acid producing strains of S. clavuligerus

Subsequent to the transfer process, where necessary protoplasts will be regenerated and the resulting bacterial cells will be cultured in a suitable growth medium. Normally colonies of cells with the desired properties will be selected by known procedures, such as for example, exploitation of differential growth requirements, antibiotic resistance or analysis of metabolites.

The invention further relates to a microbiological method comprising culturing cells produced by the above process and isolating a metabolite produced. Suitably the metabolite is a β-lactam antibiotic. Suitable methods of culture and isolation are those known in the art.

The process of the present invention may be the final stage in a strain improvement programme, or alternatively the cells produced may be subjected to further strain modifications. When the microbiological method is employed to produce a metabolite, such as clavulanic acid, a strain may be selected which produces clavulanic acid, and in which the cells thereof are adapted to improve the

- 5 -

isolation, for example by increasing the yield of clavulanic acid or decreasing or eliminating the production of other metabolites.  The invention also relates to the metabolite  produced by the microbiological method.

One aspect of the present invention provides a process for the transfer of DNA into a streptomycete which comprises entrapment of the DNA within a lipid vesicle, fusing the lipid vesicle with a protoplast of a streptomycete, and thereafter allowing the protoplast to regenerate.

The protoplasts may suitably be allowed to regenerate at high density on an appropriate regeneration medium.  Suitable regeneration media include, for example, those disclosed by H. Okanishi et al. J. Gen Microbiol., 1974, 80, 389.

Preferably the fusion of the lipid vesicle with the protoplast is mediated by the addition of polyethylene glycol to the fusion mixture.

The lipid vesicle may suitably be prepared from a pure lipid or a lipid mixture.  Preferably lipid mixtures derived from the streptomycetes are used to form the lipid vesicle.

Suitably the lipid or lipid mixture will be chosen for the ability to form very small lipid vesicles, which might be expected to exhibit less lethality in fusion reactions.  Suitable sizes include 0.01 $\mu$m to 10 $\mu$m diameter, for example 0.01 $\mu$m to 0.1 $\mu$m, 0.1 $\mu$m to 10 $\mu$m , preferably about 1 $\mu$m, such as 0.5 $\mu$m to 5 $\mu$m. The preference for small size is because the product of a lipid vesicle protoplast fusion will contain the cytoplasm of the protoplast diluted by the contents of the lipid vesicle. Metabolic stress induced by the sudden dilution could reduce the viability of the product, whereas with small lipid vesicles, less dilution occurs

The use of a buffer more closely approximating the cell contents, for example a protoplast lysate of the recipient strain may also increase survival and hence transformation frequency. Small lipid vesicles may suitably be produced by ultrasonication. However, since such treatment is liable to mechanically shear the transforming DNA, producing many short fragments, control of conditions is important.

The following Examples illustrate the process of the present invention.

In addition, in order to demonstrate unequivocally that changes in the genotypes of strains result from the process of the present invention, data are presented to demonstrate that new combinations of genes are produced which could only have arisen by the incorporation of the donor DNA. Accordingly, the following examples include experiments designed to demonstrate the following phenomena:-

1. The transformation to prototrophy of various auxotrophic requirements using DNA from nutritionally competent cells.
2. The transformation to auxotrophy of previously prototrophic strains.
3. The transformation of strains sensitive to various chemicals to resistant variants by DNA of strains previously resistant.

4. The absence of the above effects when the transforming (donor) DNA is derived from the same isolate as the recipient strain.

5. The absence of transformation after treatments designed to destroy the informational content of the donor DNA.

Example 1

Transformation of protoplasts of S. clavuligerus and S. coelicolor following PEG induced fusion with liposomes containing DNA.

Extraction of DNA was performed using the procedure of Marmur, J. Mol Biol. 3, 1961, 208-218 with the following modifications:

1. Mycelia (2-5g) harvested after 4 days growth in Liquid complete media were treated for 10 min with 5 ml of 10 mg ml$^{-1}$ Lysozyme (Sigma) in 0.25 M Tris pH8 and 3ml of 20% sucrose, which resulted in the formation of fluffy rather than lysed cells. Lysis was achieved by the addition of 3 mls of hot (90$^{O}$C) sodium dodecyl Sulphate 25%.

2. Diethylpyrocarbonate (0.01ml m$^{-1}$) (Sigma) was used in conjunction with EDTA to inhibit the action of DNA 'ases'.
   Lipids were isolated from the chloroform - iso amylalcohol phase of the DNA extraction procedure. During the extractions of some strains of . S. coelicolor the chloroform layer was coloured pink, presumably due to the presence of proto-actinorhodin. This was removed by shaking with 0.88 aqueous ammonia which converted the compound to the water soluble, blue actinorhodin. 7-Dehydrocholesterol (10 ug ml$^{-1}$) was added to the pooled chloroform extractions of both streptomycetes and 3 ml were evaporated to dryness in vacuo.

Liposome encapsulated DNA, was prepared by adding 0.1 ml of a 10 ug ml$^{-1}$ DNA solution in G. Buffer (0.015M NaCl, 0.0015 M trisodium citrate, 0.28M sucrose, 0.01M CaCl$_2$, 0.1M threonine, 0.1M histidine) to the lipid film, and spinning the flask (on a rotory evaporator,) for 10 min. The resulting liposomes were suspended in 5 ml of P buffer, D.A. Hopwood et al. J. Gen Microbiol III, 1979, 137, and shaken vigorously for a further 10 min. after which their concentration was estimated by haemocytometer count (ave 0.2X10$^7$ml$^{-1}$).

Liposomes prepared in this manner were very variable in size, their diameters ranging from about 10 to 0.1 μm with most being about 1 μm which is a little smaller than the average size of Streptomyces protoplasts.

Protoplasts were generally prepared according to the method of Hopwood et al. J. Gen Microbiol III, 1979, 137. Occasionally the mycelia used was grown on cellophane discs on CM agar rather than in liquid culture.

Liposomes and protoplasts were mixed in equal numbers and pelleted by centrifugation. PEG treatment was as for protoplast fusion of Hopwood & Wright Molec Gen Genet 162 307 - (1978). Suitable dilutions of the fusion mixture were spread on regeneration media and incubated at 25$^{\mathrm{O}}$C. Mature colonies were 'velvet replicated' onto diagnostic media. The numbers of colonies resulting on each media was used to estimate the frequency of transformation of each marker gene. In each case at least 500 colonies were tested.

The mutant strains of S. coelicolor A3(2) used were all SCP1⁻and SCP2⁻. The marker genes have been described in Bacterial Rev 37, 371-405 (1973). The strains of S. clavuligerus are all mutagenic derivatives of S. clavuligerus strain ATCC 27064, designated N in this report. The gene symbols are as follows:- hyp, trp, met, ad, auxotrophic requirements for hypoxanthine, tryptophan, methionine and adenine respectively, ant, fur resistance to antimycin and flurouracil.

UV absorbance spectra indicate that at least 50% of the DNA is left behind in the supernatant after centrifugation. A further 20% can be removed by treating the liposomes with DNA ase suggesting it is associated with the outside of the vesicle and so may be involved in transformation. Thus our final estimation of the DNA content of each liposome is between 3 and 5 times the mass of a single genome.

Control experiments included: treating the donor DNA with DNA exonuclease (Sigma type III, 15 units, 1 hour at 37°C pH5), subjecting it to consecutive heat treatment and an ultra violet irradiation (115°C 15 min, 4.5kJ); and substituting calf thymus DNA (Sigma).

Table 1 shows the frequency of phenotypic transformation of S. clavuligerus and S. coelicolor. After fusion with liposomes containing DNA, protoplasts were allowed to regenerate and form colonies which were then replica plated onto diagnostic media. The numbers of colonies arising on each selective medium was noted and used to calculate the frequency of transformation for each marker.

## TABLE 1

| DONAR DNA GENOTYPE | | | | | | | RECIPIENT PROTOPLAST GENOTYPE | | | | | | | % OF COLONIES SHOWING TRANSFORMATION TO THE INDICATED PHENOTYPE | | | | | | |
|---|---|---|---|---|---|---|---|---|---|---|---|---|---|---|---|---|---|---|---|---|
| STRAIN | hyp1 | ade1 | ant1 | trp1 | met1 | fur | STRAIN | hyp1 | ade1 | ant | trp1 | met1 | fur | HYP | AD | ANT | TRP | MET | FUR | |
| N | + | + | + | + | + | + | CL198 | − | − | − | + | + | + | 2.2+ | 10+ | 6+ | NT | NT | NT | ⎫ |
| CL7 | − | + | + | + | + | + | CL98 | + | + | + | − | − | − | 4.4− | NT | NT | 8.1+ | 7.8+ | 8+ | |
| CL98 | + | + | + | − | − | − | CL198 | − | − | − | + | + | + | 3.7− | 8.8+ | 2.2+ | 8.1− | 3.1− | 2.4+ | ⎬ S.clavuligerus |
| CL198 | − | − | − | + | + | + | CL98 | + | + | + | − | − | − | 4− | 4.8− | 9− | 8.8+ | 9.1+ | 4− | |
| CL198 | − | − | − | + | + | + | N | + | + | + | + | + | + | 4− | 3.9− | 9.9− | NT | NT | NT | |
| CL198 | − | − | − | + | + | + | CL198 | − | − | − | + | + | + | <0.2 | <0.2 | <0.2 | NT | NT | NT | ⎭ |
| | proA | argA | cysD | hisA | uraA | strA | | proA | argA | cysD | hisA | uraA | strA | PRO | ARG | CYS | HIS | URA | STR | |
| A3(2) | + | + | + | + | + | + | 2709 | − | − | − | − | − | − | $10.1^+$ | $10.2^+$ | $9.3^+$ | $9.8^+$ | $9.2^+$ | $9.2^+$ | ⎫ |
| M124 | − | − | − | + | + | + | M130 | + | + | + | − | − | − | $6.1^-$ | $5.9^-$ | $2.8^-$ | $9.8^+$ | $9.1^+$ | $8^+$ | |
| M130 | + | + | + | − | − | − | M124 | − | − | − | + | + | + | $10^+$ | $9.7^+$ | $9.9^+$ | $7.1^-$ | $4.1^-$ | $4.2^-$ | ⎬ S.coelicolor |
| 2709 | − | − | − | − | − | − | A3(2) | + | + | + | + | + | + | $4.9^-$ | $4.5^-$ | $4.7^-$ | $4.7^-$ | $4.9^-$ | $4.8^-$ | |
| 2709 | − | − | − | − | − | − | 2709 | − | − | − | − | − | − | $<0.2^+$ | $<0.2^+$ | $<0.2^+$ | $<0.2^+$ | $<0.2^+$ | $<0.2^+$ | ⎭ |

NT = Not Tested

0061253

- 12 -

## Example 2

Frequencies of transformation of S. coelicolor and
S. clavuligerus assessed after non selective isolation.
Fusion mixtures were spread at suitable dilutions to give
nearly confluent lawns of growth on regeneration media.
Spore suspensions obtained from these plates were
spread on CM to give ca 50 colonies per plate.  These
colonies were then velvet replicated onto diagnostic
media and the resulting colonies assessed for the presence
or absence of the various genetic markers.  Genotypes
of the strains are given in table 1.

# TABLE 2

| DONOR DNA | RECIPIENT PROTOPLAST | % AGE TRANSFORMATION | | | | | | |
|---|---|---|---|---|---|---|---|---|
| | | proA | hisA | argA | cysD | strA | uraA | GENOTYPE |
| M124 | M130 | −<br>9.1 | +<br>9.8 | −<br>10 | −<br>10 | +<br>9.7 | +<br>9.5 | |
| A3(2) | 2709 | +<br>10 | +<br>10.2 | +<br>9.8 | +<br>8.1 | +<br>8.1 | +<br>9.3 | S. COELICOLOR |
| 2709 | A3(2) | −<br>9.8 | −<br>10.1 | −<br>9.5 | −<br>9.3 | −<br>9.7 | −<br>9.1 | |
| | | trp 1 | met 1 | fur | hyp 1 | ad 1 | ant | GENOTYPE |
| CL98 | CL198 | −<br>8.8 | −<br>8.1 | −<br>8.0 | +<br>8.5 | +<br>8.9 | +<br>8.6 | S. CLAVULIGERUS |
| CL198 | CL98 | +<br>9.1 | +<br>8.9 | +<br>8.1 | −<br>9.5 | −<br>9.1 | −<br>9.2 | |

Example 3

Frequencies of various recombination events during three component transformations of S. coelicolor. Table 3 Section a), shows the frequencies of recombination events detected when liposomes ($2 \times 10^6$) containing DNA from strain M124 (proA argA cysD) were used to transform a mixture (1:1 ratio) of protoplasts ($2 \times 10^6$) from strains 2685 (proA uraA cysD) and 2686 (uraA, argA, cysD).

Table 3 Section b) shows the frequencies when ($2 \times 10^6$) protoplasts of strain M124 were simultaneously transformed with a mixture of ($2 \times 10^6$) liposomes containing DNA from either strain 2685 or 2686 (1:1 ratio) prepared separately.

Table 3 Section c) as section b) except that the liposomes were prepared containing a mixture of DNA from strains 2685 and 2686.

In all cases, after fusion the protoplasts were allowed to regenerate at high density on regeneration medium. Spore suspensions obtained from the resulting lawn were spread on plates of CM at a dilution sufficient to give about 100 colonies per plate. These colonies served as a source of innoculum to construct 10 master plates of 64 colonies each which were then velvet replicated onto diagnostic media for genotype analysis.

## TABLE 3 a) b) & c)

| | GENOTYPE | | | RECOMBINATION EVENT | FREQUENCY % |
|---|---|---|---|---|---|
| | cysD | proA | argA | uraA | | |

a)

| cysD | proA | argA | uraA | RECOMBINATION EVENT | FREQUENCY % |
|---|---|---|---|---|---|
| − | + | + | − | FUSION OF 2685 AND 2886 | 10.2 |
| − | − | + | + | TRANSFORMATION OF 2985 | 4.8 |
| − | + | − | + | TRANSFORMATION OF 2686 | 4.4 |
| − | + | + | + | MULTIPLE INTERACTION | 6.8 |

b)

| cysD | proA | argA | uraA | RECOMBINATION EVENT | FREQUENCY % |
|---|---|---|---|---|---|
| − | + | + | − | DOUBLE TRANSFORMATION | 0.2 |
| − | − | + | + | TRANSFORMATION BY 2685 | 9.2 |
| − | + | − | + | TRANSFORMATION BY 2686 | 9.1 |
| − | + | + | + | DOUBLE TRNSFORMATION | 0.8 |

c)

| cysD | proA | argA | uraA | RECOMBINATION EVENT | FREQUENCY % |
|---|---|---|---|---|---|
| − | + | + | − | DOUBLE TRANSFORMATION | 2.6 |
| − | − | + | + | TRANSFORMATION BY 2685 | 3.0 |
| − | + | − | + | TRANSFORMATION BY 2686 | '3.8 |
| − | + | + | + | DOUBLE TRANSFORMATION | 3.2 |

## Claims

1.      A process for the transfer of genetic material into Actinomycete cells, which comprises entrapment of the genetic material within a lipid vesicle and thereafter fusing the lipid vesicle with an osmotically fragile Actinomycete cell.

2.      A process as claimed in claim 1 wherein the osmotically fragile Actinomycete cell is a protoplast.

3.      A process as claimed in claim 1 or claim 2 wherein the genetic material is DNA.

4.      A process as claimed in claim 3 wherein the genetic material is chromosomal phage or plasmid DNA.

5.      A process as claimed in any one of claims 1 to 4 wherein fusion is promoted by treatment with polyethylene glycol.

6.      A process as claimed in claim 5 wherein the polyethylene glycol has a molecular weight of 1000 to 6000.

7.      A process as claimed in any one of claims 1 to 6 wherein the cells are of the genus Streptomyces.

8.      A process as claimed in claim 7, wherein the cells are of the species S. coelicolor, S. clavuligerus, S. olivaceous, Streptomyces P6621, S. jumonjinensis or S. lividans.

9.    A process as claimed in claim 1 for the transfer of DNA into a Streptomycete which comprises entrapment of the DNA within a lipid vesicle, fusing the lipid vesicle with a protoplast of a streptomycete, and thereafter allowing the protoplast to regenerate.

10.    Cells whenever prepared by the process of any one of claims 1 to 9.

11.    Cells as claimed in claim 10, which produce β-lactam antibiotic.

12.    Cells as claimed in claim 10 or 11 of the genus Streptomyces.

13.    Cells as claimed in claim 12 of the species S. clavuligerus, S. olivaceous, S. coelicolor, S. katsurahamanus, Streptomyces P6621, S. jumonjinensis or S. lividans.

14.    Cells as claimed in claim 13, being a strain of S. clavuligerus producing clavulanic acid.

15.    A microbiological method comprising culturing cells produced by the process of any of claims 1 to 9 and isolating a metabolite.

16.    A microbiological method as claimed in claim 15 wherein the metabolite is a β-lactam antibiotic.

17.    A microbiological method as claimed in claim 16 wherein the metabolite is clavulanic acid.

18.    Clavulanic acid whenever produced by the microbiological method as claimed in claim 17.

19.     A process as claimed in any one of claims 1 to 9
wherein the cells produced are subjected
to further strain modifications.

20.     A metabolite isolated from cells
produced by the process as claimed in claim 19.